# EUROPEAN PATENT APPLICATION

(11) **EP 4 831 998 A1**
(43) Date of publication of application: **28.10.2026**
(21) Application number: 24906843.8
(22) Date of filing: 20.09.2024
(51) Int. Cl.: G16H 20/10, G06Q 10/08, G06Q 50/26

(54) **MEDICINE ASSIST SYSTEM, MEDICINE ASSIST METHOD, AND MEDICINE ASSIST PROGRAM**

(30) Priority: 18.12.2023 JP 2023213333
(71) Applicant: Credopharmacy Co., Ltd., Kawasaki-shi, Kanagawa 214-0021 (JP)
(72) Inventor: HOMMA, Hideaki, Kawasaki-shi, Kanagawa 214-0021 (JP)
(74) Representative: AWA Sweden AB
(86) International application number: PCT/JP2024/033674
(87) International publication number: WO 2025/134453

(57) **Abstract**

[Problem] To provide is a medicine assist system that takes into account factors such as medicine regulations at a delivery destination including a foreign country and is capable of delivering a medicine even to a foreign country.

[Solution] The medicine assist system comprises: a receiving unit configured to receive information described in a prescription from a patient-side terminal; an information generation unit configured to generate dispensing data indicating a medicine based on the information described in the prescription; an audit unit configured to audit the dispensing data; and an instruction unit configured to issue an instruction to deliver a prescribed medicine including the medicine to a patient.

## Description

### [Technical field]

The present invention relates to a medicine assist system, a medicine assist method, and a medicine assist program in which a pharmacist remotely performs a dispensing operation and delivers a dispensed prescribed medicine to a patient when the patient attempts to obtain the prescribed medicine via the Internet.

### [Background art]

Conventionally, a patient brought a prescription to a pharmacy, where a pharmacist performed a dispensing operation on-site based on the prescription. With the spread of the Internet, an increasing number of pharmacists perform dispensing operations online and deliver dispensed prescribed medicines to a patient's address.

The dispensing support system disclosed in Patent Document 1 has a function of remotely auditing a dispensing operation by collating dispensing information generated based on an image of a prescription with prescription information described in the prescription, thereby making it possible to reduce a workload of a pharmacist.

Furthermore, a prescription medicine order management system disclosed in Patent Document 2 comprises: an acquisition unit configured to acquire a plurality of pieces of data including data indicating contents of a prescription; a medicine instruction data generation unit; a storage unit configured to store various data; and a medicine instruction management unit configured to manage an execution status of medicine instruction. This prescription medicine order management system acquires information indicating the contents of a prescription and application information of a patient, and generates order data. Furthermore, the prescription medicine order management system generates information such as a date and time for implementing remote medicine instruction to the patient based on the order data, and transmits the information to a pharmacist in charge. In this case, since the pharmacist in charge can provide the remote medicine instruction to the patient at the predetermined implementation date and time, it is possible to reduce a workload at a pharmacy.

### [Prior Art Documents]

### [Patent Documents]

Patent Document 1: Japanese Patent Application Publication No. 2023-107632
Patent Document 2: Japanese Patent Application Publication No. 2023-096917

### [Summary of the Invention]

### [Problems to be Solved by the Invention]

However, the systems described in Patent Document 1 and Patent Document 2 disclose only a remote dispensing operation within a country. When attempting to deliver a dispensed prescribed medicine to a foreign delivery destination, there is a possibility that the medicine cannot be delivered to a foreign country due to factors such as medicine regulations in the foreign country.

Accordingly, the present invention has been made in view of the above problems, and an object thereof is to provide a medicine assist system, a medicine assist method, and a medicine assist program that take into account factors such as medicine regulations at a delivery destination including a foreign country and enable delivery of a medicine even to a foreign country.

### [Means for solving the problem]

As one embodiment of the present invention, a medicine assist system comprises: a receiving unit configured to receive information described in a prescription from a patient-side terminal; an information generation unit configured to generate dispensing data indicating a medicine based on the information described in the prescription; an audit unit configured to audit the dispensing data; and an instruction unit configured to issue an instruction to deliver a prescribed medicine including the medicine to a patient.

In the medicine assist system, a status acquisition unit may be provided to acquire, as regulatory data, an identifier of a medicine regulated at a delivery destination to which a prescribed medicine is to be delivered, wherein the information generation unit generates the dispensing data by linking the medicine described in the prescription with the medicine identifier, and the audit unit determines that the dispensing data has validity when none of the medicine identifiers indicated in the dispensing data are included in the medicine identifiers included in the regulatory data, and when it is determined that the dispensing data has validity, the instruction unit issues the instruction to deliver. The audit unit may determine that a patient is an authorized user when information included in personal information of the patient input from the patient-side terminal completely matches information included in personal information described in the prescription input from the patient-side terminal, and when it is determined that the patient is an authorized user, the instruction unit may issue the instruction to deliver. In the medicine assist system, the system may further comprise: a computation unit configured to, when the audit unit determines that the dispensing data has validity and also determines that the patient is an authorized user, generate information including the dispensing data as passed medicine display information; and an identification code generation unit configured to link an identification code for identifying a plurality of pieces of information with the information including the passed medicine display information, and generate identification information capable of displaying information regarding the prescribed medicine by causing the patient-side terminal to identify the identification code.

A medicine assist method comprises executing, by a computer: a receiving step of receiving information described in a prescription from a patient-side terminal; an information generation step of generating dispensing data indicating a medicine based on the information described in the prescription; an audit step of auditing the dispensing data; and an instruction step of issuing an instruction to deliver a prescribed medicine including the medicine to a patient.

A medicine assist program causes a computer to implement: a receiving function of receiving information described in a prescription from a patient-side terminal; an information generation function of generating dispensing data indicating a medicine based on the information described in the prescription; an audit function of auditing the dispensing data; and an instruction function of issuing an instruction to deliver a prescribed medicine including the medicine to a patient.

### [Effects of the Invention]

According to one embodiment of the present application, the medicine assist system according to the present invention audits whether or not the prescribed medicine can be delivered to the delivery destination. Therefore, according to the present invention, it becomes possible to provide a patient with a prescribed medicine suitable for a delivery destination.

### [Brief Description of the Drawings]

FIG. 1 is a schematic diagram illustrating a configuration of a medicine assist system.
FIG. 2 is a block diagram illustrating an example of a configuration of an intermediate terminal.
FIG. 3 is a data table showing data extracted by the personal information acquisition unit.
FIG. 4 is a data table showing identification information.
FIG. 5 is a data table showing data extracted by the prescription information identification unit.
FIG. 6 is a flowchart illustrating an example of a process for generating medicine display information.
FIG. 7 is a flowchart illustrating an example of a process for generating identification information.

### [Embodiment for Carrying out the Invention]

An embodiment of a medicine assist system 10 according to the present invention will be described below with reference to the drawings. Note that the present invention is not limited to the embodiment. In addition, some components that are not important for description are omitted in the drawings.

### <Overview>

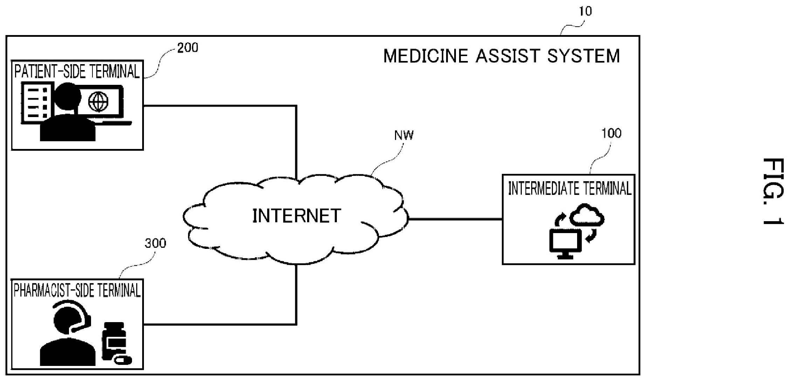

FIG. 1 is a schematic diagram illustrating a configuration of a medicine assist system 10 according to an embodiment of the present invention. In the medicine assist system 10 according to the present embodiment, an intermediate terminal 100, a patient-side terminal 200, and a pharmacist-side terminal 300 are communicably connected via the Internet NW.

The intermediate terminal 100 can receive various types of information (including, for example, information capable of identifying an individual patient, or medicine information described in a prescription) from the patient-side terminal 200, and can transmit the information to the pharmacist-side terminal 300. In addition, the intermediate terminal 100 can audit information regarding personal information of the patient transmitted from the patient-side terminal 200, and can audit various types of information (including, for example, medicine information) transmitted from the pharmacist-side terminal 300.

In the present invention, "audit" refers to determining whether or not information has validity by collating the information. Here, when there is validity, it may be indicated that a subject of the audit has no problem. In addition, the audit in the present invention may be at least one of: (i) auditing contents of a prescription; (ii) auditing whether or not an owner of a prescription who applies for a medicine is the person himself/herself; (iii) auditing whether or not a medicine prescribed based on a prescription is described at a delivery destination; (iv) auditing whether or not a medicine prescribed based on a prescription is correct; and (v) auditing whether or not a dispensing activity by a pharmacist is correct. Note that when it is determined that there is no validity in at least one of the above (i) to (v), the prescribed medicine may not be delivered to the patient. Here, as a criterion for determining whether or not a medicine has validity, that is, whether or not a medicine is regulated, various regulations that can regulate medicines in each country, such as laws, orders, ministerial ordinances, and ordinances (hereinafter referred to as "pharmaceutical regulations, for example"), are included, and for example, a law concerning import/export activities of medicines may be included. The medicine assist system 10 can perform an audit based on the pharmaceutical regulations, for example, of the patient's country which is the delivery destination. As an example, when a medicine included in a prescribed medicine is not regulated based on the pharmaceutical regulations, for example, the prescribed medicine may be delivered to the patient. Conversely, when a medicine included in a prescribed medicine is regulated based on the pharmaceutical regulations, for example, the prescribed medicine may not be delivered to the patient.

The intermediate terminal 100 may generate a patient-specific account based on information capable of identifying an individual patient (for example, it may be an insurer number, a date of birth, a gender, an email address, or a phone number, but is not limited to these) and grant the patient a right to use the medicine assist system 10. For example, a patient may input his/her personal information in the patient-side terminal 200, and the patient-side terminal 200 may transmit the input personal information to the intermediate terminal 100. The intermediate terminal 100 may generate a patient-specific account based on the patient's personal information, and then transmit the patient-specific account to the patient-side terminal 200.

In addition, the intermediate terminal 100 determines whether or not an account owner and a prescription owner are the same person by collating personal information of the account owner with various types of information described in a prescription, thereby auditing impersonation of a patient, and may audit a dispensing activity of a pharmacist (for example, whether or not the dispensing activity of the pharmacist is fraudulent).

The patient-side terminal 200 can associate information described in a prescription input by the patient (for example, the information may be an insurer number, a date of birth, a gender, a phone number, or information regarding a medicine, but is not limited to these) with the patient-specific account, and transmit the associated information to the intermediate terminal 100. In addition, a means for inputting information is not limited, and the patient may manually input various types of information described in the prescription, or may input the information by photographing the prescription and uploading the photograph to the patient-side terminal 200.

Note that a prescription according to the present invention may be acquired by a patient from a doctor at a hospital, or may be acquired from a doctor online. In addition, when the patient undergoes an online medical examination, the doctor may transmit an electronic file of the prescription (for example, a photograph of the prescription or a copy of the prescription) to an email address of the patient, or may send an original of the prescription to an address of the patient.

The pharmacist-side terminal 300 can receive an input of various types of information from a pharmacist. The pharmacist-side terminal 300 can acquire information from the intermediate terminal 100, and can acquire, for example, passed data including patient information and information regarding a medicine. The pharmacist can confirm the information regarding the medicine included in the passed data and perform a dispensing activity on the pharmacist-side terminal 300.

### <Configuration>

### <Intermediate Terminal 100>

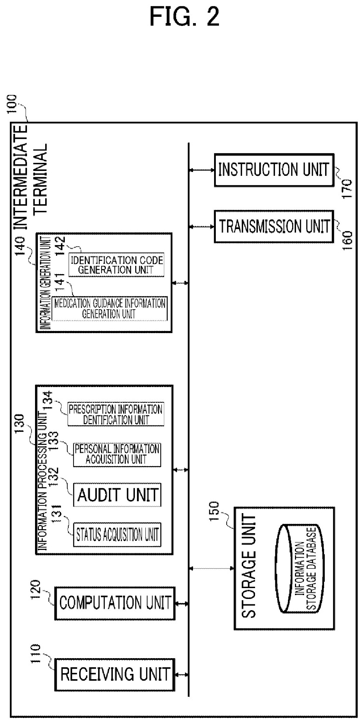

Next, an intermediate terminal 100 according to an embodiment of the present invention will be described with reference to FIG. 2. The intermediate terminal 100 may include a receiving unit 110, a computation unit 120, an information processing unit 130, an information generation unit 140, a storage unit 150, a transmission unit 160, and an instruction unit 170. The intermediate terminal 100 is typically a computer system, and implements functions using a Central Processing Unit (CPU), a Graphics Processing Unit (GPU), for example. Note that FIG. 2 is an example, and the configuration of the intermediate terminal 100 is not limited to that illustrated.

The receiving unit 110 can receive various types of information from the patient-side terminal 200 and the pharmacist-side terminal 300. The receiving unit 110 can receive, for example, the patient's personal information and the information described in the prescription from the patient-side terminal 200. In addition, various types of information received by the receiving unit 110 may be stored in the storage unit 150 as unprocessed information.

The computation unit 120 can compute various costs related to a prescription of a medicine. In addition, the computation unit 120 may add a part or all of the various costs to compute a total amount of the part or all. Here, the various costs may include a cost of a medicine described in a prescription of a patient and a cost required for delivering a prescribed medicine (hereinafter referred to as a "shipping cost"), and may include a dispensing cost due to a dispensing activity of a pharmacist and a fee for using the medicine assist system 10. In addition, the computation unit 120 may be connected to a reimbursement claim computer system so as to be communicable, and may request the reimbursement claim computer system to compute various costs (for example, medical fees). Here, the reimbursement claim computer system refers to a computer system that creates a "reimbursement claim (medical fee statement)" in order to claim medical fees to a payment organization such as a medical facility or a health insurance association.

The information processing unit 130 can include a status acquisition unit 131, an audit unit 132, a personal information acquisition unit 133, and a prescription information identification unit 134. The information processing unit 130 may have a function of determining whether or not a prescription owner is the person himself/herself, whether or not a medicine included in a prescribed medicine is regulated by overseas pharmaceutical regulations, for example, and whether or not a dispensing activity of a pharmacist follows a certain prescription.

The status acquisition unit 131 can acquire various types of information. Here, the acquired various types of information may include pharmaceutical regulations, for example, of a delivery destination of a prescribed medicine, environmental information of the delivery destination of the prescribed medicine (for example, information that may affect storage of the prescribed medicine, such as temperature, atmospheric pressure, humidity, and light irradiation), for example.

In addition, the status acquisition unit 131 may specify a medicine regulated based on the pharmaceutical regulations, for example, of the delivery destination of the prescribed medicine, and link an identifier of the medicine as regulatory data.

The identifier used as the identifier of the medicine is not limited, and the status acquisition unit 131 can assign a unique identifier to each medicine based on information capable of identifying the medicine, such as a medical medicine search number. As an example of acquiring the pharmaceutical regulations of the delivery destination, for example, when a patient in Country A transmits prescription information to a pharmacist in Country B, the status acquisition unit 131 may automatically acquire the pharmaceutical regulations, for example, of Country A, which is a country corresponding to the IP address, when the receiving unit 110 receives a transmission IP address (Internet Protocol Address) of the patient. In addition, the status acquisition unit 131 may acquire the pharmaceutical regulations, for example, of each country in advance and store them in the storage unit 150. The status acquisition unit 131 may identify the patient's IP address and directly acquire regulatory data based on the transmission IP address of the patient from the storage unit 150. In addition, along with acquiring the pharmaceutical regulations, for example, the status acquisition unit 131 may acquire an identifier of a medicine regulated based on the pharmaceutical regulations, for example, by linking the identifier as regulatory data.

The audit unit 132 can audit dispensing data indicating a medicine generated in the information generation unit 140 by collating the dispensing data with regulatory data, based on the information described in the prescription received in the receiving unit 110. Here, the dispensing data is generated by linking a medicine described in a prescription with an identifier. When at least one of the medicine identifiers indicated in the dispensing data is included in the medicine identifiers included in the regulatory data, it may be determined that the prescribed medicine cannot be delivered to the patient.

The audit unit 132 may audit a prescribed medicine itself dispensed by a pharmacist. As an audit of a prescribed medicine, for example, the audit unit 132 may audit a dispensing activity of a pharmacist. In this case, the audit unit 132 has a capturing function to capture a dispensing activity of a pharmacist, and captures and audits activities such as checking, weighing, and mixing of medicines, and checking of a medicine bag or a medicine tag when a pharmacist is dispensing. A captured moving image and a prescription may be stored in the storage unit 150 as audit data and audited by the audit unit 132, or may be transmitted as audit data to an external terminal capable of auditing a dispensing activity of a pharmacist and audited by the external terminal. The audit of a dispensing activity of a pharmacist may be to audit whether or not a dispensing activity of a pharmacist complies with a local pharmacist law. If at least any of the dispensing activities violate the pharmacist law, the audit unit 132 may determine that the dispensed prescribed medicine has no validity.

The personal information acquisition unit 133 can extract and acquire personal information of a patient from unprocessed information stored in the storage unit 150. As an example, the personal information acquisition unit 133 may acquire a patient's personal information input when creating a patient account (for example, it may include the patient's personal phone number, email address, insurer number, address, for example, but is not limited to these), and the information generation unit 140 may generate a patient-specific account based on the patient's personal information. The personal information acquisition unit 133 may acquire information capable of identifying an individual account owner (for example, it may include the patient's personal phone number, email address, insurer number, but is not limited to these) as unchangeable information, and may acquire information incapable of identifying an individual account owner (for example, the information may include a gender, a location, an age, for example, but is not limited to these) as changeable information. As a result, even when a location is changed due to a patient's moving for example, information relating to an account owner can be freely changed.

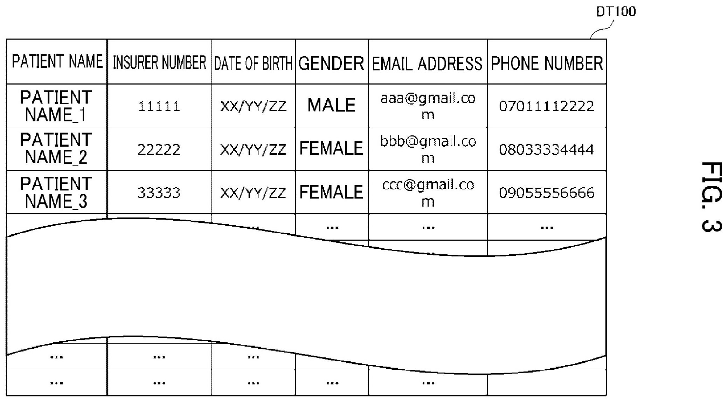

FIG. 3 is an example of a data table showing information relating to a patient-specific account generated based on personal information extracted in the personal information acquisition unit 133. The personal information acquisition unit 133 can acquire information specifying an individual account owner from personal information received in the receiving unit 110. A data table DT100 may include information such as "patient name", "insurer number", "date of birth", "gender", "email address", and "phone number", but is not limited to these.

The prescription information identification unit 134 can extract and acquire information described in a prescription from unprocessed information stored in the storage unit 150. In addition, the prescription information identification unit 134 may further identify various types of information described in a prescription, classify the various types of information into prescription owner information and medicine information, and acquire the various types of information. The prescription information identification unit 134 can, for example, identify various types of information described in a prescription, classify the various types of information into prescription owner information indicating personal information of a prescription owner and medicine information including a medicine name and a medicine dose, and acquire them. As an example, the prescription information identification unit 134 may recognize a name of a patient and an insurer number of a predetermined number of digits described in a prescription using morphological analysis, and acquire the name of a patient and the insurer number as prescription owner information. The prescription information identification unit 134 may acquire information capable of identifying an individual prescription owner (for example, the information may include the patient's personal phone number, email address, insurer number, for example, described in the prescription, but is not limited to these) as personal information of a prescription owner based on received information, and may acquire information incapable of identifying an individual prescription owner (for example, the information may include a gender, an age, but is not limited to these) as information that is not personal information of a prescription owner. In addition, medicine information may be stored in the storage unit 150 as it is.

In addition, the prescription information identification unit 134 may have a function of recognizing an image of a prescription. As an example, when an image of a prescription is uploaded, the prescription information identification unit 134 can classify information described in the prescription from the image of the prescription into prescription owner information indicating an identity of a prescription owner and medicine information including a medicine name and a medicine dose, and identify the information. For example, the prescription information identification unit 134 may identify an insurer number in an image of a prescription and acquire the number as identity information of a prescription owner. Other information that cannot identify an identity of a prescription owner may be deleted without being stored in the storage unit 150 after an image is acquired. Note that when a prescription is in a foreign language, the information processing unit 130 may further include a translation unit, and after acquisition by the prescription information identification unit 134, a foreign language portion included in a photograph of the prescription may be translated into a language understandable to a pharmacist.

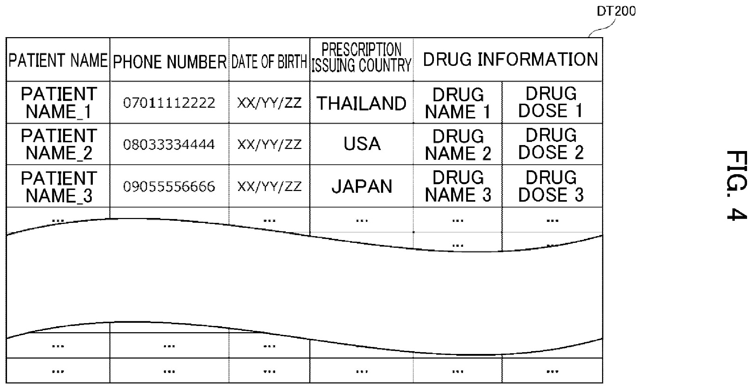

A data table DT200, which is an example of a data table showing information acquired from a prescription extracted in the prescription information identification unit 134, is shown in FIG. 4. The data table DT200 may include information described in a prescription such as "patient name", "phone number", "date of birth", "prescription issuing country", and "medicine (drug) information", but is not limited to these.

After the personal information acquisition unit 133 and the prescription information identification unit 134 acquire patient information (personal information of an account owner and personal information of a prescription owner), the audit unit 132 can audit an identity of a patient. As an example, when an account owner A inputs various types of information described in a prescription of a patient B (prescription owner) (for example, the information may include a name of patient B, an insurer number, a medicine name, a medicine dose) into the medicine assist system 10, the medicine assist system 10 may determine whether or not the account owner and the prescription owner are the same person by comparing personal information of the account owner A (for example, the personal information may include an insurer number and an insurer name) with the input personal information described in the prescription of the patient B (for example, the personal information may include an insurer number and an insurer name). In addition, the medicine assist system 10 may be connected to a database of a medical institution in a country where a patient is located. In this case, after acquiring connection permission from a medical institution in a country where the patient is located, the medicine assist system 10 searches the database of the medical institution in the country where the patient B is located using personal information of the account owner A (for example, the personal information may include an insurer number and an insurer name) as a keyword, and determines whether or not there is a prescription record of the account owner A. When there is a prescription record of the account owner A, various types of information included in the record (for example, the various types of information may include a medicine name and a medicine dose) are compared with various types of information described in the prescription of the patient B. When various types of information do not match, it may be determined that the account owner A and the patient B described in the prescription are not the same person.

When a medicine described in a prescription complies with pharmaceutical regulations, for example, and a prescription owner and an account owner are the same person, the audit unit 132 may certify dispensing data as passed data. In addition, the medicine assist system 10 is connected to a reimbursement claim computer system that is compliant with a dispensing system prescription IF shared specification (NSIPS (registered trademark): New Standard Interface of Pharmacy-system Specifications), and may transmit passed data to a dispensing auditing system, an automatic tablet/powder packaging machine, or another dispensing system to generate a shared specification for dispensing.

Note that the computation unit 120 may generate passed medicine display information by linking the computed total amount with the passed data.
The passed medicine display information may not include the total amount.

The information generation unit 140 can include a medication guidance information generation unit 141 and an identification code generation unit 142. The information generation unit 140 may generate medication guidance information and an identification code for identifying various types of information stored in the storage unit 150. The information generation unit 140 may generate dispensing data by linking a medicine described in a prescription with an identifier of the medicine.

The medication guidance information generation unit 141 can generate information relating to a prescription of a prescribed medicine. As an example, in the storage unit 150, information to be conveyed to a patient when a medicine is prescribed is stored in advance in association with a medicine included in a prescribed medicine, and when a medicine to be prescribed is identified, the medication guidance information generation unit 141 may generate information to be conveyed to a patient when the medicine is prescribed. For example, the medication guidance information generation unit 141 may generate information such as "Please take this prescribed medicine at the same time every day before or after a meal.", "Please avoid taking alcohol while taking this prescribed medicine." for example. When a pharmacist inputs precautions during prescription such as a type and dose of each medicine, efficacy of a dispensed prescribed medicine, a method of taking, medicine instruction, and precautions on the pharmacist-side terminal 300 accompanying a dispensing activity, the medication guidance information generation unit 141 may generate medication guidance information based on the information input by the pharmacist. In addition, various types of information generated in the medication guidance information generation unit 141 may be linked with passed medicine display information and generated as information to be provided to a patient. The information to be provided to a patient may not include various types of information generated in the medication guidance information generation unit 141.

Information to be provided to a patient may be displayed in a plurality of languages. In this case, the information processing unit 130 further includes a translation unit, and the translation unit can translate information to be provided to a patient into a plurality of languages. For example, when a language of information to be provided to a patient is English and a pharmacist is Japanese, the translation unit can translate the English version of the information to be provided to a patient into Japanese. Here, the obtained Japanese version of information to be provided to a patient and the English version of the information to be provided to a patient may be stored as one set. A translated sentence (that is, a sentence obtained by translating into a language of another country based on information to be provided to a patient) may be freely adjusted according to a nationality of a patient and a delivery destination of a prescribed medicine.

The identification code generation unit 142 can generate a code for identifying information to be provided to a patient, link the code with information to be provided to a patient, and generate identification information. An identification code may be a barcode (one-dimensional code) or a QR code (registered trademark) (two-dimensional code), but is not limited to these, and may be a character code. After generating an identification code, the identification code generation unit 142 may link the identification code with information to be provided to a patient. A patient can confirm information to be provided to a patient by reading an identification code.

Identification information can include patient information, medicine (drug) information, dispensing data, passed data, a total amount, passed medicine display information, medication guidance information, information to be provided to a patient, and an identification code, and a patient can acquire various types of information regarding a prescribed medicine using an identification code.

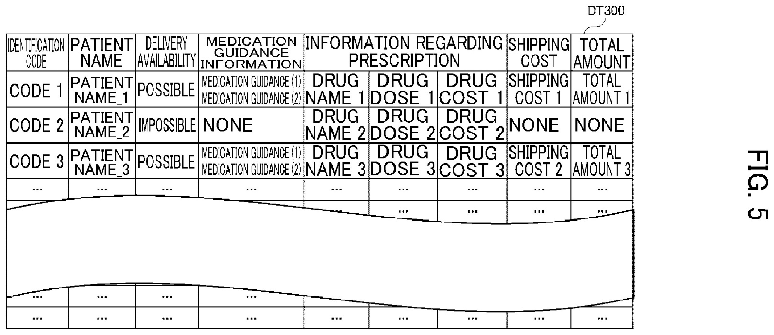

FIG. 5 shows an example of identification information generated in the identification code generation unit 142. In a data table DT300 shown in FIG. 5, as identification information, an identification code, a patient name, delivery availability, medication guidance information, information regarding a prescription, a shipping cost, and a total amount are included, but not limited to these. The identification code generated in the identification code generation unit 142 is transmitted to the patient-side terminal 200 by the transmission unit 160 and displayed on the patient-side terminal 200, whereby a patient can read the identification code on the patient-side terminal 200. As an example, when patient 1 reads code 1, which is an identification code, "Patient name_1, Delivery possible, Medication guidance (1) and Medication guidance (2), Drug name 1, Drug dose 1, Drug cost 1, Shipping cost 1 and Total amount 1" may be displayed on the patient-side terminal 200.

The storage unit 150 is a recording medium for storing various programs and various data required in the intermediate terminal 100. The storage unit 150 is embodied by, for example, a Hard Disk Drive (HDD), a Solid-State Drive (SSD), a flash memory, for example, but is not limited thereto. The storage unit 150 may include, for example, a flash memory, a Random Access Memory (RAM), a Read Only Memory (ROM), for example.

The transmission unit 160 can transmit various types of information to the patient-side terminal 200 and the pharmacist-side terminal 300. The transmission unit 160 may transmit, for example, a digital statement of delivery including identification information and a prescription to the patient-side terminal 200. As a result, a patient can confirm identification information on the patient-side terminal 200 while referring to a prescription.

The instruction unit 170 can issue a delivery instruction to the pharmacist-side terminal 300.

### <Examples of Operations and Display Screens>

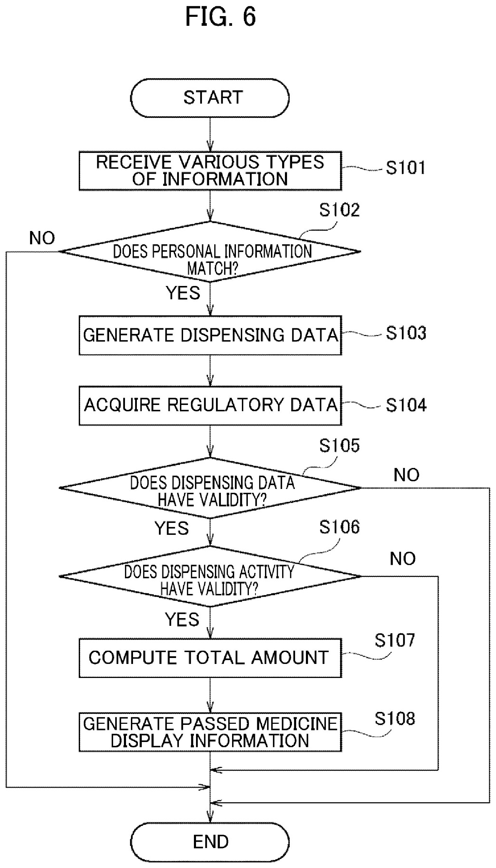

Next, an embodiment of the medicine assist system 10 according to the present invention will be described based on a flowchart and a data table. FIG. 6 is an example of a flowchart showing a part of an operation of the intermediate terminal 100, and the present invention is not limited to these.

First, various types of information are received from the patient-side terminal 200 (S101). The audit unit 132 audits whether or not personal information input by a patient from the patient-side terminal 200 matches personal information described in a prescription input to the patient (S102). When at least one of information included in the personal information input by the patient and information included in the personal information described in the prescription input to the patient does not match, the audit unit 132 determines that a patient who has input from the patient-side terminal 200 or an individual described in the prescription has no validity as an unauthorized user (NO in S102), and terminates an operation of the intermediate terminal 100. When information included in the personal information input by the patient and information included in the personal information described in the prescription input to the patient completely match, the audit unit 132 determines that a patient who has input from the patient-side terminal 200 and an individual described in the prescription are the same person and is an authorized user (YES in S102), and proceeds to a next step.

The information generation unit 140 generates dispensing data indicating a medicine based on the information described in the prescription input from the patient-side terminal 200 (S103). The status acquisition unit 131 acquires pharmaceutical regulations, for example. of a delivery destination of a prescribed medicine, recognizes and specifies a medicine to be regulated based on the pharmaceutical regulations, of the delivery destination, for example, of the prescribed medicine, and links an identifier of the medicine to link the medicine as regulatory data (S104). The audit unit 132 collates dispensing data with regulatory data and audits the dispensing data (S105). When at least one of medicine identifiers indicated in dispensing data is included in medicine identifiers included in regulatory data, the audit unit 132 determines that the dispensing data has no validity (NO in S105), and terminates an operation of the intermediate terminal 100.

When none of medicine identifiers indicated in dispensing data is included in medicine identifiers included in regulatory data, the audit unit 132 determines that the dispensing data has validity, certifies the dispensing data as passed data (YES in S105), and proceeds to a next step. The audit unit 132 audits a dispensing activity of a pharmacist (S106). When it is determined that a dispensing activity of a pharmacist has no validity (NO in S106), the audit unit 132 terminates an operation of the intermediate terminal 100. When it is determined that a dispensing activity of a pharmacist has validity (YES in S106), the audit unit 132 proceeds to a next step.

Then, the computation unit 120 computes a total amount (S107). The computation unit 120 generates passed medicine display information by linking a total amount and passed data (S108).

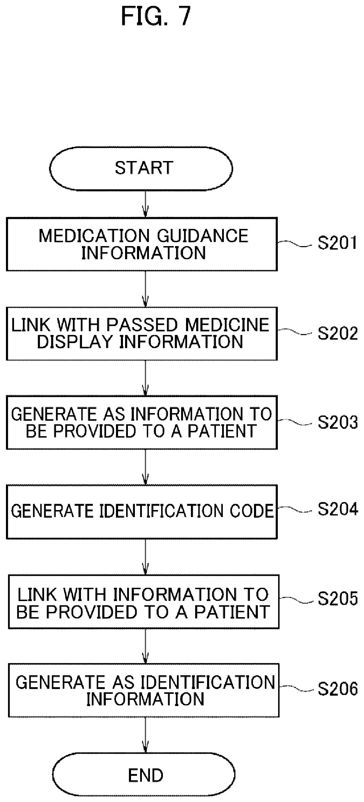

FIG. 7 is a flowchart showing a part of an operation in which the intermediate terminal 100 generates identification information. Note that FIG. 7 is an example, and the present invention is not limited to these.

First, the medication guidance information generation unit 141 generates medication guidance information relating to a dispensed prescribed medicine (S201).

Next, the medication guidance information generation unit 141 links medication guidance information generated by the medication guidance information generation unit 141 with passed medicine display information (S202), and generates the medication guidance information as information to be provided to a patient (S203).

Thereafter, the identification code generation unit 142 generates an identification code for identifying information to be provided to a patient (S204).

The identification code generation unit 142 links the generated identification code with information to be provided to a patient (S205), and generates the identification code as identification information (S206).

Here, as medication guidance information, a moving image in which a pharmacist performs medicine instruction, for example, may be included, and this moving image may be transmitted from the pharmacist-side terminal 300.

In addition, when it is determined in the audit unit 132 that there is no validity, before terminating an operation of the intermediate terminal 100, the audit unit 132 may generate information indicating that a process is interrupted, such as "Delivery impossible" or "Dispensing impossible", and transmit the information to the patient-side terminal 200. The audit unit 132 may generate information such as "Delivery impossible" or "Dispensing impossible" as request rejection information by linking it with an identification code, personal information of a patient, and information relating to a prescription. As a result, a patient can grasp a situation where a prescribed medicine cannot be acquired.

Note that when it is determined that there is no validity, a patient may be able to transmit a message requesting reconfirmation to the intermediate terminal 100. In this case, the intermediate terminal 100 may transmit a request of a patient to the pharmacist-side terminal 300. A pharmacist may reconfirm information included in identification information in response to a request of a patient on the pharmacist-side terminal 300, and may be able to perform processing again in the case of a pharmacist's mistaken response or insufficient explanation, and a pharmacist may be able to additionally write a more detailed explanation in identification information, but is not limited thereto.

Upon receiving a message indicating remittance for a prescribed medicine from the patient-side terminal 200, the instruction unit 170 transmits a command to deliver the prescribed medicine to the pharmacist-side terminal 300. At this time, information necessary for delivery may also be transmitted to the pharmacist-side terminal 300. A pharmacist may pack a prescribed medicine according to the command and then deliver the prescribed medicine to an address described in a patient-specific account.

Note that in a command to deliver a prescribed medicine, an information list relating to settlement information (for example, referred to as a statement of delivery or a receipt) and data of an information label describing delivery destination information may be transmitted to the pharmacist-side terminal 300, and a command to print these data may also be transmitted. In this case, a pharmacist may deliver a prescribed medicine, an information list, and an information label to an address of a patient in accordance with the command.

### <Supplementary Notes>

Although an embodiment of the present invention has been described, it goes without saying that the present invention is not limited to the above embodiment. Various modified examples and mountable functions will be described below.

(1) In the above embodiment, after a patient-specific account is created, the medicine assist system 10 may transmit a notification of completion of account creation to the patient-side terminal 200. As an example, after a patient creates an account, the medicine assist system 10 transmits a notification of completion of account creation to a phone number or an email address of the patient. At this time, a notification of completion of account creation may not include personal information of a patient.
(2) In the above embodiment, in the medicine assist system 10, information relating to a patient-specific account may be stored in an external server. As an example, the medicine assist system 10 may be connected to a Network Attached Storage (NAS) server. After a patient input his/her personal information, for example, into the medicine assist system 10, the medicine assist system 10 transmits the personal information to the NAS server. Thereafter, the NAS server may automatically distribute a URL of a storage destination for storing various types of information to a patient by email or short message in accordance with creation of a patient information folder. When a patient intends to reconfirm his/her personal information, after clicking a URL of a storage destination, his/her personal information may be automatically displayed. In addition, an owner of the medicine assist system 10 can manage information relating to a patient-specific account in the NAS server.
(3) In the above embodiment, the medicine assist system 10 may store a history of taking medicine of a patient. As an example, after various types of processing of the intermediate terminal 100 are executed, a history of taking medicine or medication guidance information of a patient may be stored in the storage unit 150 or the NAS server. When suffering from a same disease, a patient may search an existing history of taking medicine and take a prescribed medicine with reference to the history of taking medicine and medication guidance information. As another example, a history of taking medicine of a patient before using the medicine assist system 10 may be stored in the storage unit 150 or the NAS server. After acquiring connection permission from a medical institution in a country where a patient is located, the medicine assist system 10 is connected to a database of a medical institution in a country where a patient is located, and the medicine assist system 10 may extract a history of taking medicine of a patient from a database of a medical institution based on personal information of a patient, particularly a name and an insurer number of a patient, and store the history of taking medicine in the storage unit 150 or the NAS server. In addition, a patient may manually input his/her history of taking medicine up to now into the medicine assist system 10 and store the history of taking medicine in the storage unit 150 or the NAS server. In this case, a patient may freely confirm a history of taking medicine linked with a patient-specific account on the patient-side terminal 200.
(4) In the above embodiment, the medicine assist system 10 may be connected to a generative AI program and automatically acquire medicine instruction. As an example, when medication guidance information of a pharmacist is insufficient, a patient may acquire medication guidance information of a prescribed medicine in a question-and-answer format by conversing with the generative AI program connected to the medicine assist system 10.
(5) In the above embodiment, when the medicine assist system 10 is connected to the generative AI program, the medicine assist system 10 may generate advice for patient's health based on medication guidance information of a prescribed medicine. As an example, in the case of low back pain, the medicine assist system 10 may generate advice such as "Avoid an unreasonable posture!" or "Please manage stress properly" and transmit the advice to the patient-side terminal 200.
(6) In the above embodiment, the medicine assist system 10 may be connected to an external terminal generating an identification code. As an example, the information generation unit 140 may further include an identification code storage unit. The identification code generation unit 142 may acquire an identification code such as a QR code from an external terminal and store these identification codes in an identification code storage unit.
(7) In the above embodiment, the medicine assist system 10 may provide a telemedicine system. As an example, the patient-side terminal 200 and the pharmacist-side terminal 300 are provided with a camera function, and a pharmacist may provide a patient with explanation of dispensing and medicine instruction of a prescribed medicine via an online diagnostic.
(8) In the above embodiment, the medicine assist system 10 may further include a medical questionnaire input unit. As an example, a patient may input information described in a medical questionnaire acquired from a hospital or a pharmacy into a medical questionnaire input unit on the patient-side terminal 200. A method of inputting a medical questionnaire is not limited, and a patient may manually input information described in a medical questionnaire to a medical questionnaire input unit, or a medical questionnaire input unit further includes a text recognition display function, and after a patient takes a photograph of a medical questionnaire, by uploading the photograph to a medical questionnaire input unit, a medical questionnaire input unit may read information described in a photograph of a medical questionnaire. Since information acquired from a medical questionnaire input unit is stored in the storage unit 150, a patient may reconfirm a photograph of a medical questionnaire linked with a patient-specific account or information described in a medical questionnaire on the patient-side terminal 200.

### [Reference Numerals]

10 Medicine Assist System
100 Intermediate Terminal
200 Patient-side Terminal
300 Pharmacist-side Terminal
110 Receiving Unit
120 Computation unit
130 Information Processing Unit
131 Status Acquisition Unit
132 Audit Unit
133 Personal Information Acquisition Unit
134 Prescription Information Identification Unit
140 Information Generation Unit
141 Medication Guidance Information Generation Unit
142 Identification Code Generation Unit
150 Storage Unit
160 Transmission Unit
170 Instruction Unit

## Claims

1. A medicine assist system comprising:
a receiving unit configured to receive information described in a prescription from a patient-side terminal;
an information generation unit configured to generate dispensing data indicating a medicine based on the information described in the prescription;
an audit unit configured to audit the dispensing data;
an instruction unit configured to issue an instruction to deliver a prescribed medicine including the medicine to a patient; and
a status acquisition unit configured to acquire, as regulatory data, an identifier of a medicine regulated at a delivery destination to which the prescribed medicine is to be delivered,
wherein the information generation unit generates the dispensing data by linking the medicine described in the prescription with the medicine identifier,
the audit unit determines that the dispensing data has validity when none of the medicine identifiers indicated in the dispensing data are included in the medicine identifiers included in the regulatory data, and
when it is determined that the dispensing data has validity, the instruction unit issues the instruction to deliver the prescribed medicine.

2. The medicine assist system according to claim 1, wherein
the audit unit determines that the patient is an authorized user if information included in personal information of the patient input from the patient-side terminal completely matches information included in personal information described in the prescription input from the patient-side terminal, and if the audit unit determines that the patient is an authorized user, the instruction unit issues the instruction to deliver the prescribed medicine.

3. The medicine assist system according to claim 1, further comprising:
a computation unit configured to generate information including the dispensing data as passed medicine display information when the audit unit, which determines that the patient is an authorized user if information included in personal information of the patient input from the patient-side terminal completely matches information included in personal information described in the prescription input from the patient-side terminal, determines that the dispensing data has validity and determines that the patient is an authorized user; and
an identification code generation unit configured to link an identification code for identifying a plurality of pieces of information with the information including the passed medicine display information, and generate identification information capable of displaying information regarding the prescribed medicine by causing the patient-side terminal to identify the identification code.

4. A medicine assist method comprising executing, by a computer:
a receiving step of receiving information described in a prescription from a patient-side terminal;
an information generation step of generating dispensing data indicating a medicine based on the information described in the prescription;
an audit step of auditing the dispensing data;
an instruction step of issuing an instruction to deliver a prescribed medicine including the medicine to a patient; and
a status acquisition step of acquiring, as regulatory data, an identifier of a medicine regulated at a delivery destination to which the prescribed medicine is to be delivered,
wherein in the information generation step, the dispensing data is generated by linking the medicine described in the prescription with the medicine identifier,
in the audit step, it is determined that the dispensing data has validity when none of the medicine identifiers indicated in the dispensing data are included in the medicine identifiers included in the regulatory data, and
when it is determined that the dispensing data has validity, in the instruction step, the instruction is issued to deliver the prescribed medicine.

5. A medicine assist program for causing a computer to implement:
a receiving function of receiving information described in a prescription from a patient-side terminal;
an information generation function of generating dispensing data indicating a medicine based on the information described in the prescription;
an audit function of auditing the dispensing data;
an instruction function of issuing an instruction to deliver a prescribed medicine including the medicine to a patient; and
a status acquisition function of acquiring, as regulatory data, an identifier of a medicine regulated at a delivery destination to which the prescribed medicine is to be delivered,
wherein, by the information generation function, the dispensing data is generated by linking the medicine described in the prescription with the medicine identifier,
by the audit function, it is determined that the dispensing data has validity when none of the medicine identifiers indicated in the dispensing data are included in the medicine identifiers included in the regulatory data, and
when it is determined that the dispensing data has validity, by the instruction function, the instruction is issued to deliver the prescribed medicine.
